# EUROPEAN PATENT APPLICATION

(11) **EP 4 332 230 A1**
(43) Date of publication of application: **06.03.2024**
(21) Application number: 21939438.4
(22) Date of filing: 28.05.2021
(51) Int. Cl.: C12N 15/77, C12N 9/10, C12P 13/08

(54) **CORYNEBACTERIUM GLUTAMICUM VARIANT WITH IMPROVED L-LYSINE PRODUCTION ABILITY, AND METHOD FOR PRODUCING L-LYSINE USING SAME**

(30) Priority: 30.04.2021 KR 20210056580; 26.05.2021 KR 20210067391
(71) Applicant: CJ Cheiljedang Corporation, Seoul 04560 (KR)
(72) Inventor: HONG, In Pyo, Icheon-si Gyeonggi-do 17384 (KR); LEE, Sun Hee, Yongin-si Gyeonggi-do 16923 (KR); KIM, Ha Eun, Ulju-gun Ulsan 44932 (KR); PARK, Seok Hyun, Namyangju-si Gyeonggi-do 12258 (KR); PARK, Joon Hyun, Seongnam-si Gyeonggi-do 13523 (KR)
(74) Representative: Regimbeau
(86) International application number: PCT/KR2021/006681
(87) International publication number: WO 2022/231056

(57) **Abstract**

Provided are a *Corynebacterium glutamicum* variant with improved L-lysine producing ability and a method of producing L-lysine using the same. The variant increases or enhances the expression of a gene encoding transketolase, thereby improving a production yield of L-lysine, as compared to a parent strain.

## Description

### [Technical Field]

The present disclosure relates to a *Corynebacterium glutamicum* variant with improved L-lysine producing ability and a method of producing L-lysine using the same.

### [Background Art]

L-Lysine is an essential amino acid that is not synthesized in the human or animal body and must be supplied from the outside, and is generally produced through fermentation using microorganisms such as bacteria or yeast. In L-lysine production, naturally obtained wild-type strains or variants modified to have enhanced L-lysine producing ability thereof may be used. Recently, in order to improve the production efficiency of L-lysine, various recombinant strains or variants with excellent L-lysine producing ability and methods of producing L-lysine using the same have been developed by applying a genetic recombination technology to microorganisms such as *Escherichia coli* and *Corynebacterium,* etc., which are widely used in the production of L-amino acids and other useful substances.

According to Korean Patent Nos. 10-0838038 and 10-2139806, nucleotide sequences of genes encoding proteins including enzymes related to L-lysine production or amino acid sequences thereof are modified to increase expression of the genes or to remove unnecessary genes and thereby improve the L-lysine producing ability. In addition, Korean Patent Publication No. 1 0-2020-0026881 discloses a method of replacing the existing promoter of a gene with a promoter with strong activity in order to increase expression of the gene encoding the enzyme involved in L-lysine production.

As described, a variety of methods to increase the L-lysine producing ability are being developed. Nevertheless, since there are dozens of types of proteins such as enzymes, transcription factors, transport proteins, etc. which are directly or indirectly involved in the L-lysine production, it is necessary to conduct many studies on whether or not the L-lysine producing ability is increased according to changes in the activities of these proteins.

### [Prior Art Documents]

### [Patent Documents]

Korean Patent No. 10-0838038
Korean Patent No. 10-2139806
Korean Publication Patent No. 10-2020-0026881

### [Disclosure]

### [Technical Problem]

An object of the present disclosure is to provide a *Corynebacterium glutamicum* variant with improved L-lysine producing ability.

Further, another object of the present disclosure is to provide a method of producing L-lysine using the variant.

### [Technical Solution]

The present inventors have studied to develop a novel variant with improved L-lysine producing ability using a *Corynebacterium glutamicum* strain, and as a result, they found that L-lysine production is increased by substituting a nucleotide sequence at a specific position in a promoter of tkt gene encoding transketolase, which is involved in the L-lysine biosynthetic pathway, thereby completing the present disclosure.

An aspect of the present disclosure provides a *Corynebacterium glutamicum* variant with improved L-lysine producing ability by enhancing the activity of transketolase.

As used herein, the "transketolase" refers to an enzyme that catalyzes a reaction of producing sedoheptulose phosphate by transferring a ketol group (HOCH₂CO⁻) from xylulose phosphate to ribose phosphate in the pentose phosphate pathway. The gene encoding the transketolase consists of an operon together with genes, each encoding transaldolase, glucose-6-phosphate dehydrogenase, and 6-phosphogluconolactonase, and the gene expression of this operon is regulated by a single promoter.

According to a specific embodiment of the present disclosure, the transketolase may be derived from a strain of the genus *Corynebacterium.* Specifically, the strain of the genus *Corynebacterium* may be *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi*, or *Corynebacterium flavescens,* but is not limited thereto.

As used herein, "enhancing the activity" means that expression levels of genes encoding proteins such as target enzymes, transcription factors, transport proteins, etc. are increased by newly introducing or enhancing the genes, as compared to those of a wild-type strain or a strain before modification. Such enhancement of the activity also includes the case where activity of the protein itself is increased through substitution, insertion, or deletion of the nucleotide encoding the gene, or a combination thereof, as compared to activity of the protein originally possessed by a microorganism, and the case where the overall enzyme activity in cells is higher than that of the wild-type strain or the strain before modification, due to increased expression or increased translation of the genes encoding the same, and combinations thereof.

According to a specific embodiment of the present disclosure, enhancement of the activity of transketolase may induce a site-specific mutation in a promoter of a gene encoding transketolase.

According to a specific embodiment of the present disclosure, the promoter of the gene encoding transketolase may be represented by a nucleotide sequence of SEQ ID NO: 1.

As used herein, the "promoter" refers to a specific region of DNA that regulates gene transcription by including the binding site for RNA polymerase that initiates mRNA transcription of a gene of interest, and is generally located upstream of the transcription start site. The promoter in prokaryotes is defined as a region near the transcription start site where RNA polymerase binds, and generally consists of two short nucleotide sequences at -10 and -35 base-pair regions upstream from the transcription start site. In the present disclosure, the promoter mutation is that the promoter is improved to have high activity, as compared to a wild-type promoter, and may increase the expression of genes located downstream by inducing mutations in the promoter region located upstream of the transcription start site.

According to a specific embodiment of the present disclosure, the enhancement of the activity of transketolase may be substitution of one or more bases at -300 to -10 regions upstream from the transcription start site in the promoter sequence of the gene encoding transketolase.

More specifically, the promoter mutation of the present disclosure may be consecutive or non-consecutive substitution of one or more bases at -300 to -10 regions, preferably, consecutive or non-consecutive substitution of one, two, three, four, five, six, seven, eight, nine, or ten bases at -250 to -50 regions, -230 to -200 regions, -190 to -160 regions, or -90 to -60 regions.

According to one exemplary embodiment of the present disclosure, ccaattaacc which is a nucleotide sequence at -83 to -74 regions of the promoter sequence of tkt gene encoding transketolase of the *Corynebacterium glutamicum* strain is substituted with tgtgctgtca to obtain a *Corynebacterium glutamicum* variant having a new promoter sequence of tkt gene. Such a *Corynebacterium glutamicum* variant may include the mutated promoter sequence of tkt gene, which is represented by a nucleotide sequence of SEQ ID NO: 2.

According to one exemplary embodiment of the present disclosure, ccaattaacc which is a nucleotide sequence at -83 to -74 regions of the promoter sequence of tkt gene encoding transketolase of the *Corynebacterium glutamicum* strain is substituted with tgtggtatca to obtain a *Corynebacterium glutamicum* variant having a new promoter sequence of tkt gene. Such a *Corynebacterium glutamicum* variant may include the mutated promoter sequence of tkt gene, which is represented by a nucleotide sequence of SEQ ID NO: 3.

As used herein, the "improved production ability" means increased L-lysine productivity, as compared to that of a parent strain. The parent strain refers to a wild-type or variant strain that is a subject of mutation, and includes a subject that is directly mutated or transformed with a recombinant vector, etc. In the present disclosure, the parent strain may be a wild-type *Corynebacterium glutamicum* strain or a strain mutated from the wild-type.

According to a specific embodiment of the present disclosure, the parent strain is a variant in which mutations are induced in the sequences of genes (e.g., lysC, zwf, and horn genes) involved in the lysine production, and may be a *Corynebacterium glutamicum* strain (hereinafter referred to as *'Corynebacterium glutamicum* DS1 strain') deposited at the Korean Culture Center of Microorganisms on April 2, 2021, with Accession No. KCCM12969P.

The *Corynebacterium glutamicum* variant having the improved L-lysine producing ability of the present disclosure may include a mutated promoter sequence of the gene encoding transketolase.

According to a specific embodiment of the present disclosure, the variant may include any one of nucleotide sequences represented by SEQ ID NOS: 2 to 4 as the promoter sequence of the transketolase gene.

According to one exemplary embodiment of the present disclosure, the variant may include the promoter mutation of tkt gene encoding transketolase, thereby exhibiting the increased L-lysine producing ability, as compared to the parent strain, and in particular, may exhibit 5% or more, specifically 5% to 40%, and more specifically 10% to 30% increase in the L-lysine production, as compared to the parent strain, thereby producing 65 g to 90 g of L-lysine, preferably 70 g to 80 g of L-lysine per 1 L of the culture of the strain. Further, the variant may exhibit about 4% or more increase in the L-lysine production, as compared to the existing promoter variant of the tkt gene with a different mutation site, thereby producing L-lysine in a high yield.

The *Corynebacterium glutamicum* variant according to a specific embodiment of the present disclosure may be achieved through a recombinant vector including a variant in which part of the promoter sequence of the gene encoding transketolase in the parent strain is substituted.

As used herein, the "part" means not all of a nucleotide sequence or polynucleotide sequence, and may be 1 to 300, preferably 1 to 100, and more preferably 1 to 50, but is not limited thereto.

As used herein, the "variant" refers to a promoter variant in which one or more bases at -300 to -10 regions in the promoter sequence of the transketolase gene involved in the L-lysine biosynthesis are substituted.

According to a specific embodiment of the present disclosure, variants, each in which the nucleotide sequence at -83 to -74 regions in the promoter sequence of the transketolase gene is substituted with tgtgctgtca or tgtggtatca, may have the nucleotide sequence of SEQ ID NO: 2 or SEQ ID NO: 3, respectively.

As used herein, the "vector" is an expression vector capable of expressing a target protein in a suitable host cell, and refers to a gene construct including essential regulatory elements which are operably linked so that a gene insert is expressed. Here, "operably linked" means that a gene requiring expression and a regulatory sequence thereof are functionally linked to each other to induce gene expression, and the "regulatory elements" include a promoter for performing transcription, any operator sequence for controlling the transcription, a sequence encoding a suitable mRNA ribosome binding site, and a sequence controlling termination of transcription and translation. Such a vector may include a plasmid vector, a cosmid vector, a bacteriophage vector, a viral vector, etc., but is not limited thereto.

As used herein, the "recombinant vector" is transformed into a suitable host cell and then replicated independently of the genome of the host cell, or may be integrated into the genome itself. In this regard, the "suitable host cell", where the vector is replicable, may include the origin of replication which is a particular nucleotide sequence at which replication is initiated.

For the transformation, an appropriate technology of introducing the vector is selected depending on the host cell to express the target gene in the host cell. For example, introduction of the vector may be performed by electroporation, heat-shock, calcium phosphate (CaPO₄) precipitation, calcium chloride (CaCl₂) precipitation, microinjection, a polyethylene glycol (PEG) method, a DEAE-dextran method, a cationic liposome method, a lithium acetate-DMSO method, or combinations thereof. As long as the transformed gene may be expressed within the host cell, it may be included without limitation, regardless of whether or not the gene is inserted into the chromosome of the host cell or located outside the chromosome.

The host cells include cells transfected, transformed, or infected with the recombinant vector or polynucleotide of the present disclosure *in vivo or in vitro.* Host cells including the recombinant vector of the present disclosure are recombinant host cells, recombinant cells, or recombinant microorganisms.

Further, the recombinant vector of the present disclosure may include a selection marker, which is for selecting a transformant (host cell) transformed with the vector. In a medium treated with the selection marker, only cells expressing the selection marker may survive, and thus transformed cells may be selected. The selection marker may be represented by kanamycin, streptomycin, chloramphenicol, etc., but is not limited thereto.

The genes inserted into the recombinant vector for transformation of the present disclosure may be introduced into host cells such as microorganisms of the genus *Corynebacterium* due to homologous recombination crossover.

According to a specific embodiment of the present disclosure, the host cell may be a strain of the genus *Corynebacterium,* for example, *Corynebacterium glutamicum* strain.

Further, another aspect of the present disclosure provides a method of producing L-lysine, the method including the steps of a) culturing the *Corynebacterium glutamicum* variant in a medium; and b) recovering L-lysine from the variant or the medium in which the variant is cultured.

The culturing may be performed according to a suitable medium and culture conditions known in the art, and any person skilled in the art may easily adjust and use the medium and culture conditions. Specifically, the medium may be a liquid medium, but is not limited thereto. The culturing method may include, for example, batch culture, continuous culture, fed-batch culture, or combinations thereof, but is not limited thereto.

According to a specific embodiment of the present disclosure, the medium should meet the requirements of a specific strain in a proper manner, and may be appropriately modified by a person skilled in the art. For the culture medium for the strain of the genus *Corynebacterium,* reference may be made to a known document (Manual of Methods for General Bacteriology. American Society for Bacteriology. Washington D.C., USA, 1981), but is not limited thereto.

According to a specific embodiment of the present disclosure, the medium may include various carbon sources, nitrogen sources, and trace element components. Carbon sources that may be used include saccharides and carbohydrates such as glucose, sucrose, lactose, fructose, maltose, starch, cellulose, etc., oils and fats such as soybean oil, sunflower oil, castor oil, coconut oil, etc., fatty acids such as palmitic acid, stearic acid, and linoleic acid, alcohols such as glycerol and ethanol, and organic acids such as acetic acid. These substances may be used individually or in a mixture, but are not limited thereto. Nitrogen sources that may be used include peptone, yeast extract, meat extract, malt extract, corn steep liquor, soybean meal, urea, or inorganic compounds, e.g., ammonium sulfate, ammonium chloride, ammonium phosphate, ammonium carbonate, and ammonium nitrate. The nitrogen sources may also be used individually or in a mixture, but are not limited thereto. Phosphorus sources that may be used may include potassium dihydrogen phosphate or dipotassium hydrogen phosphate or the corresponding sodium-containing salts, but are not limited thereto. Further, the culture medium may include metal salts such as magnesium sulfate or iron sulfate, which are required for growth, but is not limited thereto. In addition, the culture medium may include essential growth substances such as amino acids and vitamins. Moreover, suitable precursors may be used in the culture medium. The medium or individual components may be added to the culture medium batchwise or in a continuous manner by a suitable method during culturing, but are not limited thereto.

According to a specific embodiment of the present disclosure, pH of the culture medium may be adjusted by adding compounds such as ammonium hydroxide, potassium hydroxide, ammonia, phosphoric acid, and sulfuric acid to the microorganism culture medium in an appropriate manner during the culturing. In addition, during the culturing, foaming may be suppressed using an antifoaming agent such as a fatty acid polyglycol ester. Additionally, to keep the culture medium in an aerobic condition, oxygen or an oxygen-containing gas (e.g., air) may be injected into the culture medium. The temperature of the culture medium may be generally 20°C to 45°C, for example, 25°C to 40°C. The culturing may be continued until a desired amount of the useful substance is produced. For example, the culturing time may be 10 hours to 160 hours.

According to a specific embodiment of the present disclosure, in the step of recovering L-lysine from the cultured variant and the medium in which the variant is cultured, the produced L-lysine may be collected or recovered from the culture medium using a suitable method known in the art depending on the culture method. For example, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity, and size exclusion), etc. may be used, but the method is not limited thereto.

According to a specific embodiment of the present disclosure, in the step of recovering lysine, the culture medium is centrifuged at a low speed to remove biomass, and the obtained supernatant may be separated through ion exchange chromatography.

According to a specific embodiment of the present disclosure, the step of recovering L-lysine may include a process of purifying L-lysine.

### [Advantageous Effects]

A *Corynebacterium glutamicum* variant according to the present disclosure may improve a production yield of L-lysine by increasing or enhancing the expression of a gene encoding transketolase, as compared to a parent strain.

### [Brief Description of the Drawing]

FIG. 1 shows a construction of a pCGI(Ptkt83-1) vector including a variation, in which a nucleotide sequence at positions -83 to -74 of a transketolase promoter is substituted with tgtgctgtca according to one exemplary embodiment of the present disclosure; and

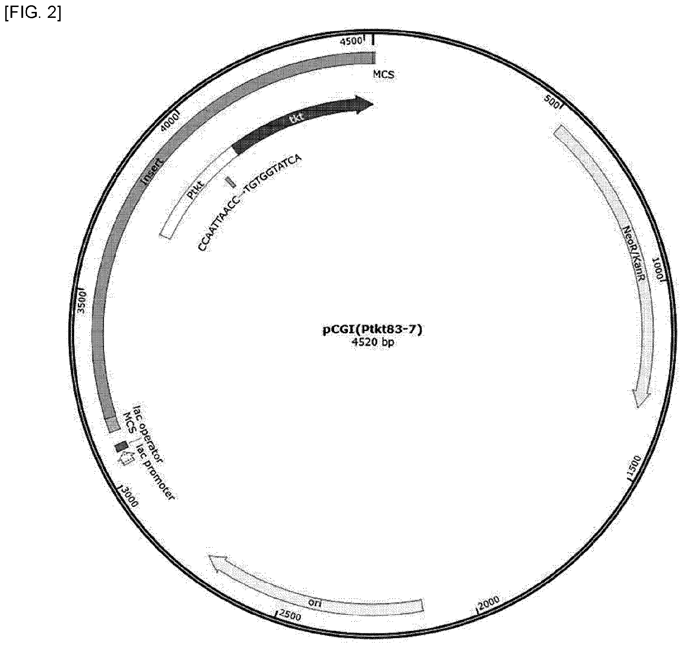

FIG. 2 shows a construction of a pCGI(Ptkt83-7) vector including a variation, in which the nucleotide sequence at positions -83 to -74 of the transketolase promoter is substituted with tgtggtatca according to one exemplary embodiment of the present disclosure.

### [Mode for Carrying Out the Invention]

Hereinafter, the present disclosure will be described in more detail. However, this description is merely provided to aid understanding of the present disclosure, and the scope of the present disclosure is not limited by this exemplary description.

### Example 1. Preparation of Corynebacterium glutamicum variant

To prepare a *Corynebacterium glutamicum* variant with the enhanced transketolase activity, *Corynebacterium glutamicum* DS1 strain was used to induce random mutation.

### 1-1. Mutagenesis

*Corynebacterium glutamicum* DS1 strain was inoculated in a flask containing 50 ml of a CM liquid medium (5 g of glucose, 2.5 g of NaCl, 5.0 g of yeast extract, 1.0 g of urea, 10.0 g of polypeptone and 5.0 g of beef extract, pH 6.8), and N-methyl-N'-nitro-N-nitrosoguanidine (NTG), which is a mutagen, was added at a final concentration of 300 µg/ml, followed by culturing at 30°C with shaking at 200 rpm for 20 hours. After completion of the culturing, the culture was centrifuged at 12,000 rpm for 10 minutes to remove the supernatant, and the resultant washed once with saline, and washed three times or more with phosphate buffer. This was suspended in 5 ml of phosphate buffer, spread on a solid medium for seed culture (15 g/l agar and 8% lysine was further added to CM liquid medium), and cultured at 30°C for 30 hours to isolate 100 colonies.

### 1-2. Selection of variants with improved L-lysine producing ability and Preparation of mutation libraries

Each 5% of 100 isolated colonies was inoculated into a flask containing 10 ml of a lysine production liquid medium shown in Table 1 below, and cultured with shaking at 200 rpm at 30°C for 30 hours. The degree of bacterial growth was confirmed by measuring absorbance of each culture at OD 610 nm, and the production of L-lysine was measured using HPLC (Shimazu, Japan). Through this, the L-lysine production was compared, and colonies producing 67.0 g/l or more of L-lysine were selected, and nucleotide sequences of lysine-related major genes thereof and promoter regions were analyzed to identify strains with mutations in the tkt gene promoter.

**[Table 1]**

| Composition | Content (based on 1 L) |
|---|---|
| Glucose | 100 g |
| Ammonium sulfate | 55 g |
| KH₂PO₄ | 1.1 g |
| MgSO₄ · H₂O | 1.2 g |
| MnSO₄ · H₂O | 180 mg |
| FeSO₄ · H₂O | 180 mg |
| Thiamine · HCl | 9 mg |
| Biotin | 1.8 mg |
| CaCO₃ | 5% |
| pH | 7.0 |

### Example 2. Improvement of tkt promoter

### 2-1. Improvement of promoter: Introduction of mutations

30 candidate sequences with up to 15 nucleotide sequence modifications covering the positions on the tkt promoter, which were selected in Example 1, were synthesized by a method of [Sambrook, J. et al. (2001) "Molecular Cloning: A Laboratory Manual", Cold Spring Harbor Laboratory Press. volume 2. 13.36-13.39], and the tkt promoter sequence of *Corynebacterium glutamicum* ATCC13032 (see SEQ ID NO: 1) and the synthesized tkt promoter region were cloned into a chloramphenicol acetyltransferase (CAT) reporter vector and a pSK1-CAT vector, respectively. During DNA cloning, orientation and the presence or absence of mutations were confirmed through DNA sequencing. The mutant libraries thus prepared were named pSK1-tkt1 to pSK1-tkt30. Finally, they were transformed into *Corynebacterium glutamicum* ATCC13032, respectively, and the promoter activities thereof were compared and examined.

### 2-2. Transformation of pSK1-CAT construct into Corynebacterium glutamicum ATCC13032

Competent cells were prepared for transformation of each of the prepared pSK-tkt clones, which were identified through sequence analysis, into *Corynebacterium glutamicum* ATCC13032. 10 ml of the cultured *Corynebacterium glutamicum* ATCC13032 was inoculated in 100 ml of a BHIS medium, and cultured at 30°C overnight, and then inoculated again in 100 ml of a CM liquid medium at OD 600 of 0.3, and cultured at 18°C and 120 rpm for about 28 hours until OD 600 became 0.8. The culture medium was centrifuged at 4°C and 6000 rpm for 10 minutes to recover the cells, which were then suspended in 20 ml of a 10% glycerol solution, and a centrifugation process was repeated three times. The recovered cells were again suspended in a 10% glycerol solution, each 100 µl was dispensed into E-tubes, and stored in a deep freezer at -70°C until use. 1 µg of DNA was added to 100 µl of *Corynebacterium glutamicum* ATCC13032 competent cells, and added to a cooled electroporation cuvette, and electroporation was performed using a Bio-Rad's MicroPulser. Immediately after pulsing, cells were recovered by adding 1 ml of a CM liquid medium pre-warmed at 46°C, reacted for 2 minutes on ice, and then incubated at 180 rpm in an incubator at 30°C. Then, 100 µl thereof was spread on a BHIS agar plate to which kanamycin (50 µg/ml) was added, and cultured in an incubator at 30°C.

### 2-3. CAT assay

Chloramphenicol acetyltransferase assay (CAT assay) of tkt promoter region variants was performed using a Shaw method (Shaw et al., 1991. Biochemistry. 30(44): 10806). Briefly, the transformed *Corynebacterium glutamicum* strain was cultured in a CM liquid medium supplemented with kanamycin (50 µg/ml), and then the cells were recovered to obtain a protein lysate. 5 µg of each protein, 0.1 M Tris-HCl buffer (pH 7.8), 0.4 mg/ml of 5,5'-dithiobis-2-nitrobenzoic acid (DTNB; Sigma D8130), 0.1 mM Acetyl CoA (Sigma A2056), and 0.1 mM chloramphenicol were added, and allowed to react at RT for 15 minutes, and absorbance was measured at OD 412 nm. Through this, Ptkt83-1 and Ptkt83-7 having improved CAT activity, as compared to the wild-type tkt promoter sequence, were selected.

In Ptkt83-1 and Ptkt83-7, the nucleotide sequence in -83 to -74 regions of the promoter sequence of the tkt gene encoding transketolase was replaced with tgtgctgtca and tgtggtatca, respectively. Thereafter, an experiment was performed using the *Corynebacterium glutamicum* DS1 strain to examine an increase in the L-lysine productivity due to promoter mutation of the tkt gene.

### Example 3. Preparation of promoter variant of Corynebacterium glutamicum DS1

To prepare a *Corynebacterium glutamicum* variant with the enhanced transketolase activity, *Corynebacterium glutamicum* DS1 strain and *E*. *coli* DH5a (HIT Competent cells^{™}, Cat No. RH618) were used.

The *Corynebacterium glutamicum* DS1 strain was cultured at a temperature of 30°C in a CM-broth medium (pH 6.8) having a composition of 5 g of glucose, 2.5 g of NaCl, 5.0 g of yeast extract, 1.0 g of urea, 10.0 g of polypeptone, and 5.0 g of a beef extract in 1 L of distilled water.

The *E*. *coli* DH5a was cultured at a temperature of 37°C in an LB medium having a composition of 10.0 g of tryptone, 10.0 g of NaCl, and 5.0 g of a yeast extract in 1 L of distilled water.

kanamycin and streptomycin, products of Sigma, were used, and DNA sequencing analysis was conducted at Macrogen Co., Ltd.

### 3-1. Preparation of recombinant vector

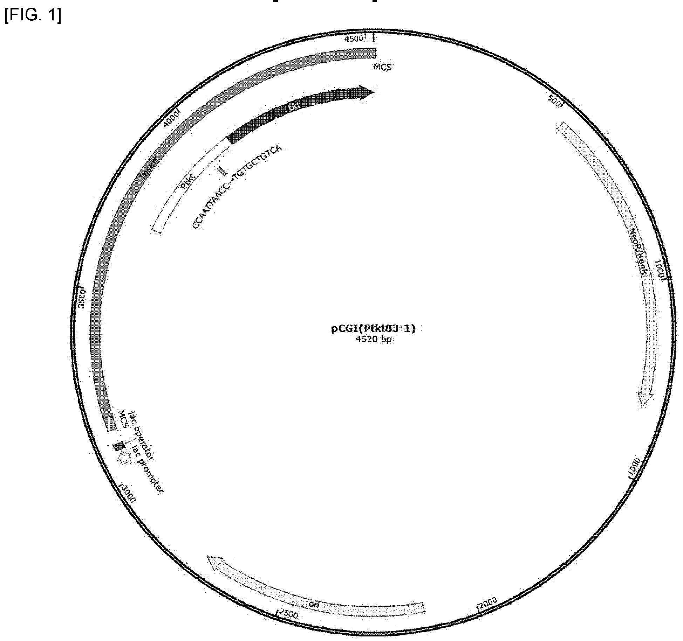

To increase lysine productivity by enhancing the activity of transketolase involved in the pentose phosphate pathway in the strain, enhancement of transketolase was introduced. The method used in this Example induced a specific mutation in a promoter of tkt gene in order to increase expression of tkt gene encoding transketolase. The nucleotide sequence at the positions -83 to -74 of the promoter of the tkt gene was replaced from ccaattaacc to tgtgctgtca, and primers containing the mutated sequences were prepared, and the 696 bp of the left arm and the 697 bp of the right arm centered around the mutated region of the tkt gene promoter on the genome of *Corynebacterium glutamicum* DS1 variant selected in Example 1 were amplified by PCR, and linked using overlap PCR, and then cloned into a recombinant vector pCGI (see [Kim et al., Journal of Microbiological Methods 84 (2011) 128-130]). The plasmid was named pCGI(Ptkt83-1) (see FIG. 1). To construct the plasmid, primers shown in Table 2 below were used to amplify each gene fragment.

**[Table 2]**

| Primer (5' - 3') | | SEQ ID NO. |
|---|---|---|
| Ptkt-1F | ggaattcccctgggcttcgttagcgc | 4 |
| Ptkt83-1-F2 | cctttgccaaatttgaatgtgctgtcataagtcgtagatctg | 5 |
| Ptkt83-1-R1 | cagatctacgacttatgacagcacattcaaatttggcaaagg | 6 |
| Ptkt-R2 | gggatccctcacgacgagcagccatgg | 7 |

The details are as follows: PCR was performed using the genomic DNA of *Corynebacterium glutamicum* DS1 strain and the corresponding primers under the following conditions. 25 to 30 cycles were performed using a thermocycler (TP600, TAKARA BIO Inc., Japan) in the presence of 1 unit of pfu-X DNA polymerase mix (Solgent, Korea) using 1 pM of oligonucleotide and 10 ng of chromosomal DNA of *Corynebacterium glutamicum* DS1 strain as a template in a reaction solution to which 100 µM of each deoxynucleotide triphosphate (dATP, dCTP, dGTP, dTTP) was added. PCR was performed under conditions of (i) denaturation step: at 94°C for 30 seconds, (ii) annealing step: at 58°C for 30 seconds, and (iii) extension step: at 72°C for 1 minute to 2 minutes (polymerization time of 2 minutes per 1 kb).

Each gene fragment thus prepared was cloned into the pCGI vector using BamHI and EcoRI restriction enzymes and a DNA Ligation kit Ver2.1 (Takara, Japan). The vector was transformed into *E*. *coli* DH5a, plated on an LB-agar plate containing 50 µg/mL kanamycin, and cultured at 37°C for 24 hours. The finally formed colonies were isolated, and it was confirmed whether the insert was correctly present in the vector, which was isolated and used in the recombination of *Corynebacterium glutamicum* strain.

### 3-2. Preparation of variant

DS1-Ptkt83-1 strain which is a strain variant was prepared using the pCGI(Ptkt83-1) vector. The vector was prepared at a final concentration of 1 µg/µL or more, and primary recombination was induced in the *Corynebacterium glutamicum* DS1 strain using electroporation (see a document [Tauch et al., FEMS Microbiology letters 123 (1994) 343-347]). At this time, the electroporated strain was then spread on a CM-agar plate containing 20 µg/µL kanamycin to isolate colonies, and then it was confirmed through PCR and base sequencing analysis whether the vector was properly inserted into the induced position on the genome. To induce secondary recombination, the isolated strain was inoculated into a CM-agar liquid medium containing streptomycin, cultured overnight or longer, and then spread on an agar medium containing the same concentration of streptomycin to isolate colonies. After examining kanamycin resistance in the finally isolated colonies, it was confirmed through base sequencing analysis whether mutations were introduced into the tkt gene in strains without antibiotic resistance (see a document [Schafer et al., Gene 145 (1994) 69-73]). Finally, *Corynebacterium glutamicum* variant (DS1-Ptkt83-10), into which the mutated tkt gene was introduced, was obtained.

### Example 4. Preparation of Corynebacterium glutamicum variant

A *Corynebacterium glutamicum* variant was prepared in the same manner as Example 3, except that the nucleotide sequence at the positions -83 ~ -74 of the promoter of the tkt gene was replaced from ccaattaacc to tgtggtatca.

Here, to construct the plasmid, primers shown in Table 3 below were used to amplify each gene fragment, and DS1-Ptkt83-7 strain which is a strain variant was prepared using the prepared plasmid pCGI(Ptkt83-7) vector. Finally, *Corynebacterium glutamicum* variant (DS1-Ptkt83-7), into which the mutated tkt gene was introduced, was obtained.

**[Table 3]**

| Primer (5' - 3') | | SEQ ID NO. |
|---|---|---|
| Ptkt-1F | ggaattcccctgggcttcgttagcgc | 4 |
| Ptkt83-7-F2 | cctttgccaaatttgaatgtggtatcataagtcgtagatctg | 8 |
| Ptkt83-7-R1 | cagatctacgacttatgataccacattcaaatttggcaaagg | 9 |
| Ptkt-R2 | gggatccctcacgacgagcagccatgg | 7 |

### Comparative Example 1. Corynebacterium glutamicum variant

A variant (Ptkt217) disclosed in Korean Patent No. 10-1504900 was used, in which the sequence at the positions -217 ~ -206 of the promoter of the tkt gene of the wild-type *Corynebacterium glutamicum* strain was replaced from ATTGATCACACC to CCCTGACTACAAA.

### Experimental Example 1. Comparison of L-lysine productivity between variants

The L-lysine productivity was compared between the parent strain *Corynebacterium glutamicum* DS1 strain, DS1-Ptkt83-1 and DS1-Ptkt83-7 strains which are the lysine producing variants prepared in Examples 3 to 4, and Ptkt217 strain of Comparative Example 1 which is the existing lysine producing variant, in the same manner as in Example 1-2. The results are shown in Table 4 below.

**Table 4**

| Strain | L-lysine (g/L) |
|---|---|
| Parent strain (DS1) | 64.2 |
| Variant (Ptkt217) | 69.9 |
| Variant (DS1-Ptkt83-1) | 73.0 |
| Variant (DS1-Ptkt83-7) | 75.1 |

As shown in Table 4, it was confirmed that *Corynebacterium glutamicum* variants, DS1-Ptkt83-1 and DS1-Ptkt83-7 strains, exhibited about 14% and 17% increases in the L-lysine productivity, respectively, as compared to the parent strain *Corynebacterium glutamicum* DS1 strain, due to substitution of the specific positions (-83 ~ -74 regions) in the promoter sequence of the tkt gene with the optimal nucleotide sequence for strengthening the lysine biosynthetic pathway.

It was also confirmed that *Corynebacterium glutamicum* variants, DS1-Ptkt83-1 and DS1-Ptkt83-7 strains, exhibited about 4% and 7% increases in the L-lysine productivity, respectively, as compared to the existing variant Ptkt217 having the different mutation site (-217 to -206 regions) and sequence (CCCTGACTACAAA).

These results indicate that the enhanced expression of the tkt gene due to mutation in the -83 to -74 regions of the promoter may promote the lysine biosynthesis ability, thereby improving the L-lysine producing ability of the strain.

Hereinabove, the present disclosure has been described with reference to preferred exemplary embodiments thereof. It will be understood by those skilled in the art to which the present disclosure pertains that the present disclosure may be implemented in modified forms without departing from the essential characteristics of the present disclosure. Accordingly, exemplary embodiments disclosed herein should be considered in an illustrative aspect rather than a restrictive aspect. The scope of the present disclosure is shown not in the aforesaid explanation but in the appended claims, and all differences within a scope equivalent thereto should be interpreted as being included in the present disclosure.

## Claims

1. A *Corynebacterium glutamicum* variant with improved L-lysine producing ability by enhancing the activity of transketolase.

2. The *Corynebacterium glutamicum* variant of claim 1, wherein the enhancing of the activity of transketolase is inducing a site-specific mutation in a promoter of a gene encoding transketolase.

3. The *Corynebacterium glutamicum* variant of claim 2, wherein the gene encoding transketolase is represented by a nucleotide sequence of SEQ ID NO: 1.

4. The *Corynebacterium glutamicum* variant of claim 1, wherein the variant comprises any one of nucleotide sequences represented by SEQ ID NOS: 2 and 3.

5. A method of producing L-lysine, the method comprising the steps of:
a) culturing the variant of claim 1 in a medium; and
b) recovering L-lysine from the variant or the medium in which the variant is cultured.
